# EUROPÄISCHE PATENTSCHRIFT

(11) **EP 1 558 185 B1**
(45) Veröffentlichungstag und Bekanntmachung des Hinweises auf die Patenterteilung: **23.05.2018**
(21) Anmeldenummer: 03769410.6
(22) Anmeldetag: 17.10.2003
(51) Int. Cl.: A61F 5/56, A61M 16/00, A61B 7/00, A61B 5/087

(54) **VERFAHREN UND VORRICHTUNG ZUR DURCHFÜHRUNG EINER SIGNALVERARBEITENDEN BETRACHTUNG EINES MIT DER ATMUNGSTÄTIGKEIT EINER PERSON IM ZUSAMMENHANG STEHENDEN MESSSIGNALES**
METHOD AND DEVICE FOR CARRYING OUT A SIGNAL-PROCESSING VIEWING OF A MEASUREMENT SIGNAL THAT IS CORRELATED TO THE RESPIRATORY ACTIVITY OF AN INDIVIDUAL
PROCÉDÉ ET DISPOSITIF DE MISE EN OEUVRE D'UNE OBSERVATION À TRAITEMENT DE SIGNAUX D'UN SIGNAL DE MESURE EN RAPPORT AVEC L'ACTIVITÉ RESPIRATOIRE D'UN INDIVIDU

(30) Priorität: 17.10.2002 DE 10248590
(43) Veröffentlichungstag der Anmeldung: 03.08.2005
(73) Patentinhaber: ResMed R&D Germany GmbH, 82152 Martinsried (DE)
(72) Erfinder: MADAUS, Stefan, 82166 Gräfelfing (DE); MEIER, Jörg, 81547 München (DE); HEIDMANN, Dieter, Castle Hill, NSW 2154 (AU); SCHNEIDER, Hartmut, Lutherville, MD 21093 (US)
(74) Vertreter: Vossius & Partner Patentanwälte Rechtsanwälte mbB
(86) Internationale Anmeldenummer: PCT/EP2003/011524
(87) Internationale Veröffentlichungsnummer: WO 2004/034938

(56) Entgegenhaltungen:
- WO-A-02/47747
- WO-A-03/059158
- US-A- 5 803 066
- US-A- 6 105 575
- US-B1- 6 363 933

## Beschreibung

Die Erfindung betrifft eine Vorrichtung zur Durchführung einer signalverarbeitenden Betrachtung eines mit der Atmungstätigkeit, beispielsweise dem Atemgasfluss im Zusammenhang stehenden Messsignales, insbesondere zur Abstimmung der Druckregelung bei der Verabreichung eines atembaren Gases auf einem zumindest phasenweise über dem Umgebungsdruck liegenden Druckniveau, sowie allgemein zur Diagnose und oder Therapie schlafbezogener Atmungsstörungen.

Zur Behandlung schlafbezogener Atmungsstörungen ist es bekannt, einem Patienten ein Atemgas, insbesondere Umgebungsluft auf einem Druckniveau zuzuführen, das zumindest phasenweise über dem Umgebungsdruckniveau liegt. Durch die Verabreichung des Atemgases auf Überdruckniveau wird es möglich, im Bereich der oberen Atemwege eine pneumatische Schienung herbeizuführen, wodurch auf physiologisch gut verträgliche Weise etwaigen Obstruktionen in diesem Atemwegsbereich vorgebeugt werden kann.

Eine besonders gute Verträglichkeit der Atemgaszufuhr auf erhöhtem Druckniveau wird erreicht, indem der Atemgasdruck auf einen möglichst geringen, lediglich zur Obstruktionsprävention, oder Obstruktionsbegrenzung hinreichenden Druck eingestellt wird. Es ist bekannt, durch eine in ein CPAP-Gerät integrierte elektronische Druckregeleinrichtung, die Einstellung des momentan erforderlichen Atemgasdruckes unter Berücksichtigung der Auswertungsresultate einer signalverarbeitenden Betrachtung des momentanen Atemgasstromes vorzunehmen. Der momentane Atemgasstrom kann insbesondere durch Volumenstrom-Sensoren z.B. Messblenden erfasst werden.

Bei CPAP-Geräten mit automatischer Druckabstimmung ist die elektronische Druckregelungseinrichtung mit der Zielsetzung konfiguriert, dass der erforderliche Atemgasdruck mit hinreichender Sicherheit bereitgestellt wird, andererseits jedoch die Dynamik der Druckvariation derart niedrig ist, dass durch die Änderungen des Atemgasdruckes der Schlafverlauf des Patienten nicht merklich beeinträchtigt wird. Beeinträchtigungen können insbesondere dann auftreten, wenn temporär vergleichsweise hohe Atemgasdruckpegel eingestellt werden.

Die US 6,363,933 B1 beschreibt Methoden und Vorrichtungen, um das Auftreten einer Apnoe, einer Durchgängigkeit und/oder einer partiellen Obstruktion der Atemwege zu bestimmen. Ein Atemluft-Fluss eines Patienten wird gemessen, um ein Atemluft-Signal zu erzeugen. Die Bestimmung einer Apnoe wird durchgeführt, indem die Varianz des Atemluft-Signals über ein gleitendes Zeitfenster berechnet und mit einem Schwellenwert verglichen wird.

Die US 6,105,575 A betrifft ein System mit Methoden und Vorrichtungen zur Behandlung einer medizinischen Störung wie obstruktiver Schlafapnoe oder kongestiver Herzinsuffizienz. Das System beinhaltet die Anwendung separater und unabhängiger Verstärkungs-Werte auf Druck-Gas, das einem Patienten während Inspirations- und Exspirationsphase eines Atemzyklus zugeführt wird, um das Druck-Gas proportional zu den jeweiligen Verstärkungs-Werten der Inspiration und Exspiration zuzuführen.

Im Dokument US 5,803,066 A werden in der Behandlung von obstruktiver Schlafapnoe ein Verfahren und eine Methode offenbart zur Detektion obstruktiver Schlafapnoe und zur Optimierung des kontrollierten positiven Drucks, um den Luft-Fluss aus einem Fluss-Generator zu minimieren während weiterhin sichergestellt wird, dass keine Fluss-Limitierung in den Atemwegen des Patienten auftritt.

Die WO 02/47747 A1 beschreibt Methoden und Vorrichtungen für die Beurteilung des Zustands und die Behandlung von Hirnschlag-Patienten während der Zufuhr von kontinuierlich positivem Atemwegsdruck (CPAP). Eine solche Methode zur Evaluierung des Zustands eines Patienten nach einem Hirnschlag beinhaltet die Schritte, atembares Gas bei einem Druck über Atmosphärendruck einem Patienten nach einem Hirnschlag zuzuführen, den Luft-Fluss dieses Patienten zu messen, und einen Hirnschlag-Indikator aus diesem Luft-Fluss zu bestimmen, wobei der Hirnschlag-Indikator Informationen über den Zustand des Patienten repräsentiert.

Der Erfindung liegt die Aufgabe zugrunde, Lösungen bereitzustellen, durch welche eine mit hoher Wahrscheinlichkeit zutreffende elektronische Auswertung eines für die Atmungstätigkeit repräsentativen Signales erreicht werden kann, so dass basierend auf dieser Auswertung der physiologische Zustand eines Patienten zutreffend bestimmt werden kann, und/oder die Atemgaszufuhr, insbesondere der Atemgasdruck verbessert auf die momentanen physiologischen Bedürfnisse abgestimmt werden kann.

Diese Aufgabe wird erfindungsgemäß dadurch gelöst, dass im Rahmen einer signalverarbeitenden Betrachtung eines für den Atemgasstrom indikativen Messsignals, Betrachtungsresultate gewonnen werden, die eine Differenzierung zwischen obstruktiven und zentralen Atmungsstörungen ermöglichen.

Dadurch wird es auf vorteilhafte Weise möglich, im Zusammenhang mit der Erfassung des momentanen Atemgasstromes eine Trendanalyse durchzuführen durch welche die für die Behebung oder Prävention einer momentanen oder bevorstehenden Atmungsstörung geeignetsten Maßnahmen, insbesondere Abstimmung der Druckregelungscharakteristik, getroffen werden können oder der Druck entsprechend eingestellt werden kann.

Gemäß der Erfindung erfolgt die signalverarbeitende Betrachtung derart, dass hierdurch die Inspirationszeit und die Exspirationszeit für abfolgende Atemzüge erfasst wird. Durch Ermittlung des Verhältnisses von Inspirationszeit und Exspirationszeit und Betrachtung der zeitlichen Änderung dieser Verhältnisse wird ein Trend erkannt, ob bevorstehende, oder bereits vorliegende Atmungsstörungen obstruktiv und/oder zentral bedingt sind.

Insbesondere in Kombination mit dieser Maßnahme ist es auch möglich, aus einer vergleichenden Betrachtung sukzessive auftretender Änderungen von Eigenschaften der Ableitungen, insbesondere der ersten Ableitung des Atemgasstromes im Bereich des Atemphasenwechsels Aussagen für eine bestehende oder bevorstehenden Störungsphase zu gewinnen.

Das Verhältnis von Inspirationszeit Ix zu Exspirationszeit Ex wird zur Beschreibung von Atmungsstörungen herangezogen.

Insbesondere kann ein Trend in der Änderung der Dauer der Inspirationszeit gegenüber der Exspirationszeit einen Hinweis auf eine bevorstehende Obstruktion in den oberen Atemwegen geben. Darüber hinaus kann die Betrachtung des Verhältnisses von Inspirationszeit Iₓ zu Exspirationszeit Eₓ in einer Trendanalyse dazu beitragen, obstruktive von zentralen Apnoen zu differenzieren.

Besonders vorteilhaft ist exakte Messung des Atemgasstromes " Flow-Kurve"

Das Verhältnis von Inspiration zu Exspiration kann als Duty-Cycle bezeichnet werden und stellt einen Informationsträger zur Bewertung der Atemflussstörungen in den oberen Atemwegen dar. Treten tatsächlich Flusslimitationen auf, so verlängert sich scheinbar die Inspirationszeit. Der nasal gemessene Widerstand (Resistance) der oberen Atemwege bleibt dagegen unverändert. Geht man davon aus, dass das Atemminutenvolumen konstant bleibt, so lässt sich ein Zusammenhang zwischen dem Volumenfluss, der Inspirationsdauer und der Atemzugdauer herleiten. (Das Atemminutenvolumen ist gleich dem Volumenfluss multipliziert mit der Inspirationszeit und dividiert durch die Atemzugdauer.)

Insbesondere in Kombination mit dieser Maßnahme, oder auch alternativ hierzu, ist es auch möglich, aus einer vergleichenden Betrachtung sukzessive auftretender Änderungen von Eigenschaften der Ableitungen der - oder innerhalb der - Atemzyklen, insbesondere der ersten Ableitung des Atemgasstromes im Bereich des Atemphasenwechsels, Aussagen für eine bestehende oder bevorstehenden Störungsphase zu gewinnen.

Die Betrachtung des Differentials kann sich auf den Beginn des Inspirationszyklus und/oder auf das Ende des Inspirationszyklus sowie auch auf die Betrachtung der Kurvenform während des Inspirationszyklus richten.

Die durchschnittliche Steigung kann in einfacher Form für Intervalle berechnet werden die sich zum Beispiel über 10% der Zeitdauer der jeweiligen Atemphase erstrecken.

Die Steigung (z.B maximale Steigung beim Phasenwechsel) kann auch innerhalb eines Fensters gleitend über den Inspirationszyklus errechnet werden.

Die Trendanalyse insbesondere hinsichtlich der Beschaffenheit und Konstitution des Atemantriebes erfolgt vorzugsweise unter Berücksichtigung/Einbeziehung der nachfolgend angegebenen Signalauswertungergebnisse:
max. Spitzenflow während des Inspirationszyklus
des Atemzugvolumes
der Inspirationszeit
der zweiten Ableitung der gemessenen Flow-Kurve

Gemäß einem weiteren Aspekt der Erfindung erfolgt die signalverarbeitende Betrachtung auf Grundlage einer Betrachtung des Differentials zu Beginn des Exspirationszyklus bzw. am Ende des Exspirationszyklus. Das Differential kann in einfacher Form über ein Intervall von z.B. 10% zu Beginn des Exspirationszyklus und nach dem exspiratorischen Maximal-Flows oder gleitend berechnet über den Exspirationszyklus errechnet werden. Die Auswertung kann ähnlich wie vorangehend beschrieben in vorteilhafter Weise unter Einbeziehung des max. Spitzenflows während des Exspirationszyklus, dem Atemzugvolumens, und/oder der Exspirationszeit und/oder die zweite Ableitung (Krümmung) der gemessenen Flow-Kurve während des Exspirationszyklus durchgeführt werden. Die Auswertung ermöglicht ebenfalls eine Aussage über die Beschaffenheit und die Konstitution der oberen Atemwege.

Die Abflachung der Atemflusskurve während des Inspirationszyklus kann nach dem Modell des Starling-Resistors als Flow-Limitation interpretiert werden. Die Betrachtung des Verlaufes der Kurvenform während des Inspirationszyklus in einem Intervall zwischen z.B. 10% nach Beginn und 10% vor Ende des Inspirationszyklus kann in vorteilhafter Weise Aufschluss über die elastischen Eigenschaften der oberen Atemwege geben. Ein Rückschluss auf den Pcrit-Wert (Druck, bei dem die oberen Atemwege verschließen) kann hierdurch ebenfalls gezogen werden.

Die Signalverarbeitung umfasst in vorteilhafter Weise insbesondere die Analyse der Anzahl lokaler Maxima und Minima, der Amplitude der lokalen Maxima und Minima, der Abfolge der Größe der Amplituden lokaler Maxima und Minima sowie der Frequenz in der Abfolge der lokalen Maxima und Minima.

Die erfindungsgemäße Signalverarbeitung umfasst gemäß einem weiteren Aspekt der vorliegenden Erfindung vorzugsweise auch die spektrale und amplitudenmäßige Betrachtung eines Schnarchsignals und kann hierauf basierend Aufschluss über die Beschaffenheit der elastischen Eigenschaften der oberen Atemwege geben sowie evtl. über Art und Ort des Verschlusses in den oberen Atemwegen.

Gemäß einem besonderen Aspekt erfolgt die signalverarbeitende Auswertung und die hierauf basierende Trendanalyse auf Grundlage einer kombinierten Betrachtung wenigstens zweier nachfolgend angegebener Parameter. Die Trendanalyse basiert vorzugsweise auf einer Betrachtung der Veränderung der Verhältnisse der kombiniert betrachteten Parameter.
Inspirationszeit
Exspirationszeit
Atemzugdauer und Atemzugfrequenz
Atemzugvolumen während des Inspirationszyklus,
Atemzugvolumen während des Exspirationszyklus,
erstes Differential und zweites Differential des Atemflusses,
Amplituden lokaler Maxima und lokaler Minima,
Frequenz lokaler Maxima und lokaler Minima,
Wendepunke,
maximaler inspiratorischer Fluss und maximaler Exspiratiorischer Fluss

Die signalverarbeitende Betrachtung der oben angegebenen Parameter kann Aufschluss geben über:
- die Beschaffenheit der oberen Atemwege geben u.a. zur Differenzierung zwischen zentralen und obstruktiven Apnoen,
- die elastischen Eigenschaften der oberen Atemwege (Rückstell-Modul, E-Modul)
- den Ort einer Obstruktion
- den Schweregrades einer Schlafapnoe
- den Pcrit-Wert

Weitere Einzelheiten und Merkmale ergeben sich aus der nachfolgenden Beschreibung in Verbindung mit der Zeichnung. Es zeigen:
- **Fig.1**: einen Ausschnitt aus einem Atemgas-Fluss Diagramm zur Erläuterung einersignalverarbeitenden Betrachtung auf Grundlage einer Ermittlung der Verhältnisse der Inspirationsdauer zur Exspirationsdauer für abfolgende Atemzyklen;
- **Fig.2**: einen Ausschnitt aus einem Atemgas-Fluss Diagramm zur Erläuterung einer signalverarbeitenden Betrachtung auf Grundlage einer Betrachtung der Änderung von Kurvenformmerkmalen von abfolgenden Inspirationszyklen;
- **Fig. 3**: einen Ausschnitt aus einem Atemgas-Fluss Diagramm zur Erläuterung einersignalverarbeitenden Betrachtung auf Grundlage einer Betrachtung der Änderung von Kurvenformmerkmalen von Abfolgenden Exspirationszyklen;
- **Fig. 4**: einen Ausschnitt aus einem Atemgas-Fluss Diagramm zur Erläuterung einersignalverarbeitenden Betrachtung auf Grundlage einer Auswertung von Kurvenformmerkmalen innerhalb von Intervallen in abfolgenden Inspirationszyklen.

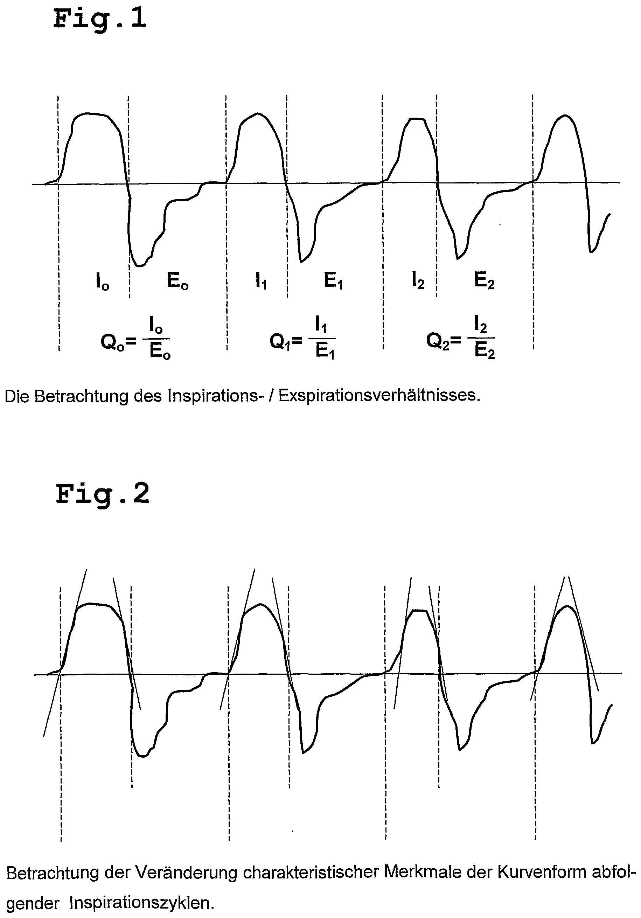

Figur 1 zeigt einen Ausschnitt aus einem Atemgas-Fluss Diagramm zur Erläuterung einer signalverarbeitenden Betrachtung auf Grundlage einer Ermittlung der Verhältnisse der Inspirationsdauer zur Exspirationsdauer für abfolgende Atemzyklen.

Das Verhältnis von Inspirationszeit Ix zu Exspirationszeit Ex und insbesondere die Veränderung desselben für abfolgende Atemzyklen, stellt eine für das Auftreten von Atmungsstörungen indikative Information dar. Insbesondere kann ein Trend in der Änderung der Dauer der Inspirationszeit gegenüber der Exspirationszeit einen Hinweis auf eine bevorstehende Obstruktion in den oberen Atemwegen geben. Darüber hinaus trägt die Betrachtung des Verhältnisses von Inspirationszeit Iₓ zu Exspirationszeit Eₓ in einer Trendanalyse dazu bei, obstruktive von zentralen Apnoen zu differenzieren. Vorteilhaft ist eine möglichst exakte Messung das Atemgasflusses und damit mögliche Abbildung der Flow-Kurve.

Das Verhältnis von Inspiration zu Exspiration kann als Duty-Cycle bezeichnet werden und stellt einen Informationsträger zur Bewertung der Atemflussstörungen in den oberen Atemwegen dar. Treten tatsächlich Flusslimitationen auf, so verlängert sich scheinbar die Inspirationszeit. Die nasal gemessene Resistance der oberen Atemwege bleibt dagegen unverändert.

Geht man davon aus, dass das Atemminutenvolumen konstant bleibt, so lässt sich ein Zusammenhang zwischen dem Volumenfluss, der Inspirationsdauer und der Atemzugdauer herleiten. (Das Atemminutenvolumen ist gleich dem Volumenfluss multipliziert mit der Inspirationszeit und dividiert durch die Atemzugdauer.)

Figur 2 zeigt einen Ausschnitt aus einem Atemgas-Fluss Diagramm zur Erläuterung einer signalverarbeitenden Betrachtung auf Grundlage einer Betrachtung der Änderung von Kurvenformmerkmalen von abfolgenden Inspirationszyklen. In dem Diagramm nach Figur 2 ist die über die erste Ableitung des Atemgasflusses ermittelte mittlere Steigung zu Beginn des Inspirationszyklus bzw. am Ende des Inspirationszyklus dargestellt. Diese mittlere Steigung ist in einfacher Form berechnet über z.B. ein 10%-Intervall oder gleitend berechnet über den Inspirationszyklus. Als weitere Kurvenformmerkmale können insbesondere die Extremwerte des Atemgasflusses (Spitzenflow während des Inspirationszyklus) und/oder das Atemzugvolumes und/oder die Inspirationszeit und/oder die zweite Ableitung der erfassten Flow-Kurve berücksichtigt werden. Die Auswertung dieser Kurvenformmerkmale - und insbesondere die Betrachtung der Veränderung derselben ermöglicht Aussagen über die Beschaffenheit und die Konstitution des Atemantriebes d.h. den momentanen -oder in Kürze vorherrschenden physiologischen Zustand des Patienten.

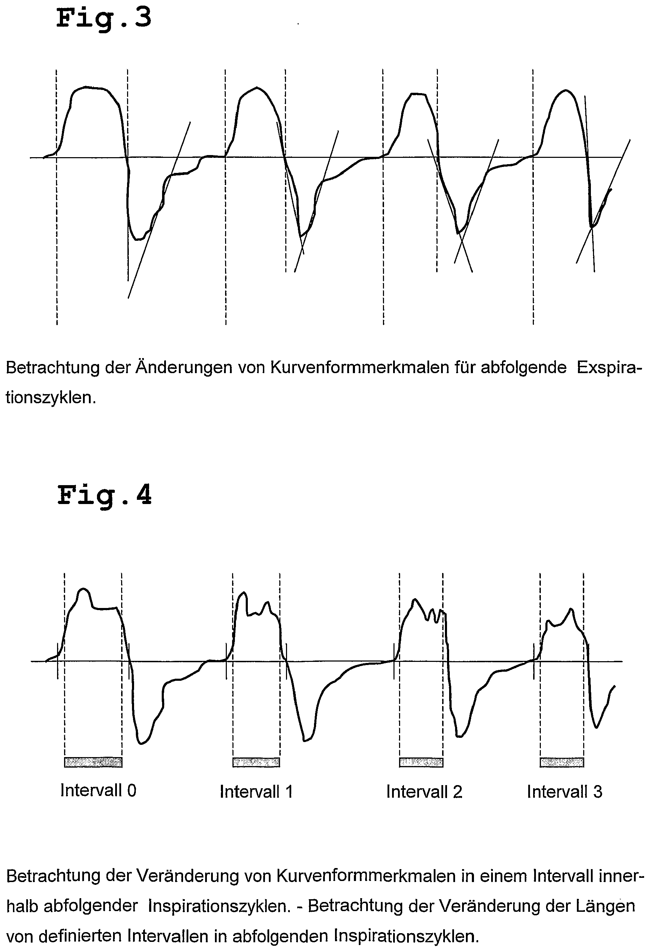

Figur 3 zeigt einen Ausschnitt aus einem Atemgas-Fluss Diagramm zur Erläuterung einer signalverarbeitenden Betrachtung auf Grundlage einer Betrachtung der Änderung von Kurvenformmerkmalen von Abfolgenden Exspirationszyklen, insbesondere in Form einer Auswertung des Differentials zu Beginn des Exspirationszyklus bzw. am Ende des Exspirationszyklus wie es in einfacher Form z.B. für ein 10%-Intervall zu Beginn des Exspirationszyklus und nach dem Exspiratorischen Maximal-Flows oder
gleitend über den Exspirationszyklus ermittelt werden kann.

Ähnlich wie für Figur 2 angegeben, können auch hier als weitere Kurvenformmerkmale insbesondere die Extremwerte des Atemgasflusses (Spitzenflow während des Exspirationszyklus) und/oder das Atemzugvolumes und/oder die Exspirationszeit und/oder die zweite Ableitung der erfassten Flow-Kurve berücksichtigt werden. Die Auswertung dieser Kurvenformmerkmale - und insbesondere die Betrachtung der Veränderung derselben ermöglicht Aussagen über die Beschaffenheit und die Konstitution des Atemantriebes d.h. den momentanen -oder in Kürze vorherrschenden physiologischen Zustand des Patienten.

Figur 4 zeigt einen Ausschnitt aus einem Atemgas-Fluss Diagramm zur Erläuterung einer signalverarbeitenden Betrachtung auf Grundlage einer Auswertung von Kurvenformmerkmalen innerhalb von Intervallen in abfolgenden Inspirationszyklen.

Die Abflachung der Atemflusskurve während des Inspirationszyklus kann (nach dem Modell des Starling-Resistors) als Flow-Limitation interpretiert werden kann. Die Betrachtung des Verlaufes der Kurvenform während des Inspirationszyklus in einem Intervall zwischen z.B. 10% nach Beginn und 10% vor Ende des Inspirationszyklus gibt Aufschluss z.B. über die elastischen Eigenschaften der oberen Atemwege.

Ein Rückschluss auf den Pcrit-Wert (Druck, bei dem die oberen Atemwege verschließen) ist durch diese Analyse ebenfalls möglich.

Bei der Durchführung einer Trendanalyse werden in vorteilhafter Weise insbesondere die folgenden Auswertungszwischenergebnisse berücksichtig:
- Anzahl lokaler Maxima und Minima
- die Amplitude der lokalen Maxima und Minima,
- die Abfolge der Größe der Amplituden lokaler Maxima und Minima sowie
- die Frequenz in der Abfolge der lokalen Maxima und Minima.
- Kurvenform in einem Intervall während des Inspirationszyklus
- Länge der Intervalle

Die spektrale und amplitudenmäßige Betrachtung eines Schnarchsignals kann weiterhin Aufschluss über die Beschaffenheit der elastischen Eigenschaften der oberen Atemwege geben sowie über Art und Ort des Verschlusses in den oberen Atemwegen.

Die Erfindung ist nicht auf die vorangehend beschriebenen Anwendungsbeispiele beschränkt. Sie kann insbesondere bei der Steuerung des Atemgasdruckes sowie der Abstimmung der Druckregelung bei einem CPAP-Gerät durch Einsatz einer entsprechend konfigurierten Signalverarbeitungseinrichtung Anwendung finden. Sie kann auch bei der zeitlich versetzten Auswertung einer Messdatenreihe Anwendung finden und ermöglicht hierbei eine Visualisierung obstruktiv oder zentral verursachter Phasen gestörter Atmung. Die Erfindung kann auch in Verbindung mit einem Pneumotachographen allgemein zur Untersuchung der Schlaf-Atmung eines Patienten herangezogen werden, ohne dass hierbei unmittelbar durch druckerhöhte Atemgaszufuhr etwaige Störungen obstruktiver Art simultan behoben werden müssen.

## Patentansprüche

1. Vorrichtung zur Durchführung einer signalverarbeitenden Betrachtung eines mit der Atmungstätigkeit einer Person im Zusammenhang stehenden Messsignals, mit einer Fördereinrichtung zur Förderung des Atemgases, einer Messeinrichtung zur Generierung eines hinsichtlich des Atemgasflusses indikativen Signales, einer Regeleinrichtung zur Einregelung des Atemgasdruckes auf einen vorgegebenen Solldruck, einer Druckvorgabeeinrichtung zur Vorgabe des Solldruckes, und mit einer Signalverarbeitungseinrichtung, die derart konfiguriert ist, dass sie im Rahmen der signalverarbeitenden Betrachtung des für den Atemgasstrom indikativen Messsignals Auswertungsresultate generiert, die eine Differenzierung zwischen obstruktiven und zentralen Atmungsstörungen ermöglichen,
**dadurch gekennzeichnet, dass**
die Inspirationszeit (Ix) und die Exspirationszeit (Ex) für abfolgende Atemzüge erfasst wird, wobei
das Verhältnis von Inspirationszeit (Ix) und Exspirationszeit (Ex) erfasst wird, wobei durch eine Betrachtung der zeitlichen Änderung dieser Verhältnisse ein Auswertungssignal generiert wird, das Aussage darüber gibt, ob bevorstehende oder bereits vorliegende Atmungsstörungen obstruktiv und/oder zentral bedingt sind, wobei
aus einer vergleichenden Auswertung sukzessive auftretender Änderungen von Eigenschaften der ersten Ableitung des Atemgasstromes im Bereich des Atemphasenwechsels Auswertungsresultate generiert werden, die darüber Aussage geben, ob eine Atmungsstörungsphase bevorsteht.

2. Vorrichtung nach Anspruch 1, **dadurch gekennzeichnet, dass** das Verhältnis von Inspirationszeit (Ix) zu Exspirationszeit (Ex) zur Beschreibung von Atmungsstörungen herangezogen wird.

3. Vorrichtung nach wenigstens einem der Ansprüche 1 bis 2, **dadurch gekennzeichnet, dass** eine Änderung der Dauer der Inspirationszeit (Ix) gegenüber der Exspirationszeit (Ex) einen Hinweis auf eine bevorstehende Obstruktion in den oberen Atemwegen darstellt.

4. Vorrichtung nach wenigstens einem der Ansprüche 1 bis 3, **dadurch gekennzeichnet, dass** aus einer vergleichenden Betrachtung sukzessive auftretender Änderungen von Eigenschaften der Ableitungen der - oder innerhalb der - Atemzyklen, insbesondere der ersten Ableitung des Atemgasstromes im Bereich des Atemphasenwechsels, Auswertungsresultate für eine bestehende oder bevorstehende Störungsphase extrahiert werden.

5. Vorrichtung nach wenigstens einem der Ansprüche 1 bis 4, **dadurch gekennzeichnet, dass** die Betrachtung der ersten Ableitung des Atemgasstromes sich auf den Beginn des Inspirationszyklus und/oder auf das Ende des Inspirationszyklus richtet.

6. Vorrichtung nach wenigstens einem der Ansprüche 1 bis 5, **dadurch gekennzeichnet, dass** sich die Betrachtung der ersten Ableitung des Atemgasstromes sich auf die Kurvenform während des Inspirationszyklus richtet.

7. Vorrichtung nach wenigstens einem der Ansprüche 1 bis 6, **dadurch gekennzeichnet, dass** die erste Ableitung des Atemgasstromes für Intervalle berechnet wird, die sich zum Beispiel über 10 % der Zeitdauer der jeweiligen Atemphase erstrecken.

8. Vorrichtung nach wenigstens einem der Ansprüche 1 bis 7, **dadurch gekennzeichnet, dass** die erste Ableitung des Atemgasstromes innerhalb eines Fensters gleitend über den Inspirationszyklus errechnet wird.

9. Vorrichtung nach wenigstens einem der Ansprüche 1 bis 8, **dadurch gekennzeichnet, dass** eine Trendanalyse hinsichtlich der Beschaffenheit und Konstitution des Atemantriebes erfolgt.

10. Vorrichtung nach wenigstens einem der Ansprüche 1 bis 9, **dadurch gekennzeichnet, dass** eine Trendanalyse unter Berücksichtigung/ Einbeziehung der nachfolgend angegebenen Signalauswertungergebnisse:
max. Spitzenflow während des Inspirationszyklus
des Atemzugvolumens
der Inspirationszeit (Ix)
der zweiten Ableitung der gemessenen Flow-Kurve
erfolgt.

11. Vorrichtung nach wenigstens einem der Ansprüche 1 bis 10, **dadurch gekennzeichnet, dass** die erste Ableitung des Atemgasstromes über ein Intervall von z.B. 10% zu Beginn des Exspirationszyklus und nach dem exspiratorischen Maximal-Flow oder gleitend berechnet über den Exspirationszyklus errechnet wird.

12. Vorrichtung nach wenigstens einem der Ansprüche 1 bis 11, **dadurch gekennzeichnet, dass** die Auswertung unter Einbeziehung des max. Spitzenflows während des Exspirationszyklus, des Atemzugvolumens, und/oder der Exspirationszeit (Ex) und/oder der zweiten Ableitung (Krümmung) der gemessenen Flow-Kurve während des Exspirationszyklus durchgeführt wird.

13. Vorrichtung nach wenigstens einem der Ansprüche 1 bis 12, **dadurch gekennzeichnet, dass** auf Grundlage der Auswertung ein Auswertungsresultat generiert wird, das Aussage über die Beschaffenheit und die Konstitution der oberen Atemwege gibt.

14. Vorrichtung nach wenigstens einem der Ansprüche 1 bis 13, **dadurch gekennzeichnet, dass** eine Betrachtung des Verlaufes der Kurvenform erfolgt, welche die Analyse der Anzahl lokaler Maxima und Minima, der Amplitude der lokalen Maxima und Minima, der Abfolge der Größe der Amplituden lokaler Maxima und Minima sowie der Frequenz in der Abfolge der lokalen Maxima und Minima umfasst.

15. Vorrichtung nach wenigstens einem der Ansprüche 1 bis 14, **dadurch gekennzeichnet, dass** die Signalverarbeitung auch die spektrale und amplitudenmäßige Betrachtung eines Schnarchsignals umfasst.

16. Vorrichtung nach wenigstens einem der Ansprüche 1 bis 15, **dadurch gekennzeichnet, dass** die signalverarbeitende Auswertung und die hierauf basierende Trendanalyse auf Grundlage einer kombinierten Betrachtung wenigstens zweier nachfolgend angegebener Parameter erfolgt: Inspirationszeit, Exspirationszeit, Atemzugdauer, Atemzugfrequenz, Atemzugvolumen während des Inspirationszyklus, Atemzugvolumen während des Exspirationszyklus, erstes Differential und zweites Differential des Atemflusses, Amplituden lokaler Maxima und lokaler Minima, Frequenz lokaler Maxima und lokaler Minima, Wendepunke, maximaler inspiratorischer Fluss und maximaler exspiratiorischer Fluss.

17. Vorrichtung nach wenigstens einem der Ansprüche 1 bis 16, **dadurch gekennzeichnet, dass** eine Trendanalyse auf einer Betrachtung der Veränderung der Verhältnisse wenigstens zweier der nachfolgend angegebenen Parameter basiert: Inspirationszeit (Ix), Exspirationszeit (Ex), Atemzugdauer, Atemzugfrequenz, Atemzugvolumen während des Inspirationszyklus, Atemzugvolumen während des Exspirationszyklus, erstes Differential und zweites Differential des Atemflusses, Amplituden lokaler Maxima und lokaler Minima, Frequenz lokaler Maxima und lokaler Minima, Wendepunkte, maximaler inspiratorischer Fluss und maximaler exspiratorischer Fluss.

18. Vorrichtung nach wenigstens einem der Ansprüche 1 bis 17, **dadurch gekennzeichnet, dass** auf Grundlage der Auswertung Auswertungsresultate generiert werden, die Aussage geben über:
- die Beschaffenheit der oberen Atemwege u.a. zur Differenzierung zwischen zentralen und obstruktiven Apnoen,
- die elastischen Eigenschaften der oberen Atemwege (Rückstell-Modul, E-Modul)
- den Ort einer Obstruktion
- den Schweregrad einer Schlafapnoe
- den Pcrit-Wert.

19. Vorrichtung nach einem der vorhergehenden Ansprüche, wobei die Signalverarbeitungseinrichtung auf Grundlage einer Veränderung von atemzyklusspezifischen Referenzmerkmalen ein Auswertungsresultat generiert, das indikativ ist, ob oder inwieweit eine vorherrschende oder bevorstehende Atmungsstörung obstruktiven oder zentralen Ursprungs ist und wobei der Solldruck unter Berücksichtigung dieses Auswertungsresultates bestimmt wird.

20. Vorrichtung nach Anspruch 19, **dadurch gekennzeichnet, dass** diese eine sich zwischen der Fördereinrichtung und einer Atemmaske erstreckende Atemgasleitung und eine Atemmaskeneinrichtung umfasst.

## Claims

1. Device for carrying out a signal-processing observation of a measuring signal which is associated with the respiratory activity of a person, having a conveying device for conveying the respiratory gas, a measuring device for generating a signal indicative with respect to the respiratory gas flow, a regulating device for regulating the respiratory gas pressure to a predefined setpoint, a pressure preset device for presetting the setpoint pressure and a signal processing device which is configured such that it generates an evaluation result within the context of the signal-processing observation of the measuring signal indicative of the respiratory gas flow, which allows a differentiation between obstructive and central respiratory disorders,
**characterized in that**
the inspiratory time (Ix) and the expiratory time (Ex) are recorded for successive breaths, wherein the relationship between inspiratory time (Ix) and expiratory time (Ex) is recorded, wherein
an evaluation signal is generated by an observation of the temporal change of this relationship which provides information as to whether impending or already existing respiratory disorders are obstructive and/or central, wherein
evaluation results are generated from a comparative evaluation of successively occurring changes in the properties of the first derivative of the flow of respiratory gas, in the region of the respiratory phase change, which provide information as to whether a respiratory disorder phase is impending.

2. Device according to claim 1, **characterised in that** the relationship between inspiratory time (Ix) to expiratory time (Ex) is used to describe respiratory disorders.

3. Device according to at least one of claims 1 to 2, **characterised in that** a change in the duration of the inspiratory time (Ix) with respect to the expiratory time (Ex) indicates an impending obstruction in the upper respiratory tract.

4. Device according to at least one of claims 1 to 3, **characterised in that** evaluation results for an existing or impending disorder phase are extracted from a comparative observation of successively occurring changes in the properties of derivatives of the - or within the - respiratory cycles, in particular of the first derivative of the flow of respiratory gas in the region of the respiratory phase change.

5. Device according to at least one of claims 1 to 4, **characterised in that** the observation of the first derivative of the flow of respiratory gas is based on the beginning of the inspiratory cycle and/or the end of the inspiratory cycle.

6. Device according to at least one of claims 1 to 5, **characterised in that** the observation of the first derivative of the flow of respiratory gas is based on the waveform during the inspiratory cycle.

7. Device according to at least one of claims 1 to 6, **characterised in that** the first derivative of the flow of respiratory gas is calculated for intervals extending, for example, over 10% of the duration of the respective respiratory phase.

8. Device according to at least one of claims 1 to 7, **characterised in that** the first derivative of the flow of respiratory gas within a window is movingly calculated over the inspiratory cycle.

9. Device according to at least one of claims 1 to 8, **characterised in that** a trend analysis, with respect to the state and constitution of the respiratory drive takes place.

10. Device according to at least one of claims 1 to 9, **characterised in that** a trend analysis takes place, taking into account/including the following signal evaluation results:
maximum peak flow during the inspiratory cycle
the respiratory volume
the inspiratory time (Ix)
the second derivative of the measured flow curve.

11. Device according to at least one of claims 1 to 10, **characterised in that** the first derivative of the flow of respiratory gas is computed over an interval of, for example, 10%, at the beginning of the expiratory cycle and after the expiratory maximum flow or movingly calculated over the expiratory cycle.

12. Device according to at least one of claims 1 to 11 , **characterised in that** the evaluation is performed taking into account the maximum peak flow during the expiratory cycle, the respiratory volume and/or the expiratory time (Ex) and/or the second derivative (curvature) of the measured flow curve during the expiratory cycle.

13. Device according to at least one of claims 1 to 12 , **characterised in that** an evaluation result is generated based on the evaluation which provides information on the state and the constitution of the upper respiratory tract.

14. Device according to at least one of claims 1 to 13 , **characterised in that** an observation of the gradient of the wave form takes place, which includes the analysis of the number of local maxima and minima, the amplitude of local maxima and minima, the sequence of the magnitudes of local maxima and minima and the frequency in the sequence of the local maxima and minima.

15. Device according to at least one of claims 1 to 14, **characterised in that** the signal processing also includes the spectral and amplitude-based observation of a snoring signal.

16. Device according to at least one of claims 1 to 15, **characterised in that** the signal-processing evaluation and the trend analysis based thereon are performed based on a combined observation of at least two of the following parameters: inspiratory time, expiratory time, respiratory duration, respiratory frequency, respiratory volume during the inspiratory cycle, respiratory volume during the expiratory cycle, first differential and second differential of the respiratory flow, amplitudes of local maxima and local minima, frequency of local maxima and local minima, inflection points, maximum inspiratory flow and maximum expiratory flow.

17. Device according to at least one of claims 1 to 16, **characterised in that** a trend analysis is based on an observation of the change in relationships of at least two of the following parameters: inspiratory time (Ix), expiratory time (Ex), respiratory duration, respiratory frequency, respiratory volume during the inspiratory cycle, respiratory volume during the expiratory cycle, first differential and second differential of the respiratory flow, amplitudes of local maxima and local minima, frequency of local maxima and local minima, inflection points, maximum inspiratory flow and maximum expiratory flow.

18. Device according to at least one of claims 1 to 17, **characterised in that** evaluation results are generated based on the evaluation which provide information on:
- the state of the upper respiratory tract, inter alia, concerning the differentiation between central and obstructive apnoeas,
- the elastic properties of the upper respiratory tract (reset module, e-module),
- the location of an obstruction,
- the severity of a sleep apnoea,
- the Perit value.

19. Device according to any one of the preceding claims, wherein the signal processing device generates an evaluation result based on a change of respiratory cycle-specific reference features, which is indicative of whether or to what extent a prevailing or impending respiratory disorder is obstructive or central in origin, and in that the setpoint pressure is determined taking into account this evaluation result.

20. Device according to claim 19, **characterised in that** it comprises a respiratory gas line extending between the supply device and a respiratory mask and a respiratory mask device.

## Revendications

1. Dispositif de mise en oeuvre d'une observation à traitement de signaux d'un signal de mesure en rapport avec l'activité respiratoire d'un individu, avec un système d'acheminement pour l'acheminement du gaz respiratoire, un système de mesure pour la génération d'un signal indicatif relatif au débit de gaz respiratoire, un système de réglage pour le réglage de la pression de gaz respiratoire à une valeur théorique prédéterminée, un système de spécification de pression pour la spécification de la pression théorique et un système de traitement de signal qui est configuré de telle sorte qu'il génère, dans le cadre de l'observation à traitement de signaux du signal de mesure indicatif du courant de gaz respiratoire, des résultats d'analyse qui permettent une différenciation entre des troubles respiratoires obstructifs et centraux,
**caractérisé en ce que**
le temps d'inspiration (Ix) et le temps d'expiration (Ex) sont saisis pour des respirations successives, dans lequel
le rapport du temps d'inspiration (Ix) et du temps d'expiration (Ex) est saisi, dans lequel, à travers une observation de la modification temporelle de ces rapports, est généré un signal d'analyse qui renseigne sur le fait que des troubles respiratoires préexistants ou déjà présents sont ou non d'origine obstructive et/ou centrale, dans lequel
à partir d'une analyse comparative de modifications apparaissant successivement de propriétés de la première dérivée du courant de gaz respiratoire dans la zone du changement de phases respiratoires, des résultats d'analyse sont générés, qui renseignent sur le fait qu'une phase de trouble respiratoire préexiste ou non.

2. Dispositif selon la revendication 1, **caractérisé en ce que** le rapport du temps d'inspiration (Ix) au temps d'expiration (Ex) est utilisé pour la description de troubles respiratoires.

3. Dispositif selon au moins l'une des revendications 1 à 2, **caractérisé en ce qu'**une modification de la durée du temps d'inspiration (Ix) par rapport au temps d'expiration (Ex) représente un indice d'une obstruction préexistante dans les voies respiratoires hautes.

4. Dispositif selon au moins l'une des revendications 1 à 3, **caractérisé en ce qu'**à partir d'une observation comparative de modifications apparaissant successivement de propriétés des dérivées des cycles respiratoires, ou à l'intérieur de ceux-ci, en particulier de la première dérivée du courant de gaz respiratoire dans la zone du changement de phases respiratoires, des résultats d'analyse pour une phase de trouble existante ou préexistante sont extraits.

5. Dispositif selon au moins l'une des revendications 1 à 4, **caractérisé en ce que** l'observation de la première dérivée du courant de gaz respiratoire s'oriente sur le début du cycle d'inspiration et/ou sur la fin du cycle d'inspiration.

6. Dispositif selon au moins l'une des revendications 1 à 5, **caractérisé en ce que** l'observation de la première dérivée du courant de gaz respiratoire s'oriente sur la forme de courbe pendant le cycle d'inspiration.

7. Dispositif selon au moins l'une des revendications 1 à 6, **caractérisé en ce que** la première dérivée du courant de gaz respiratoire est calculée pour des intervalles qui s'étendent par exemple sur 10 % de la durée de chaque phase respiratoire.

8. Dispositif selon au moins l'une des revendications 1 à 7, **caractérisé en ce que** la première dérivée du courant de gaz respiratoire est calculée à l'intérieur d'une fenêtre de façon glissante sur le cycle d'inspiration.

9. Dispositif selon au moins l'une des revendications 1 à 8, **caractérisé en ce qu'**une analyse de tendance s'effectue concernant la nature et la constitution de la pulsion respiratoire.

10. Dispositif selon au moins l'une des revendications 1 à 9, **caractérisé en ce qu'**une analyse de tendance s'effectue en prenant en compte/intégrant les résultats d'analyse de signal indiqués ci-après :
débit de pointe max. pendant le cycle d'inspiration
le volume respiratoire
le temps d'inspiration (Ix)
la deuxième dérivée de la courbe de flux mesurée.

11. Dispositif selon au moins l'une des revendications 1 à 10, **caractérisé en ce que** la première dérivée du courant de gaz respiratoire est calculée sur un intervalle par exemple de 10 % au début du cycle d'expiration et après le débit maximal expiratoire ou calculée de façon glissante sur le cycle d'expiration.

12. Dispositif selon au moins l'une des revendications 1 à 11, **caractérisé en ce que** l'analyse est réalisée en prenant en compte le débit de pointe max. pendant le cycle d'expiration, le volume respiratoire et/ou le temps d'expiration (Ex) et/ou la deuxième dérivée (courbure) de la courbe de débit mesurée pendant le cycle d'expiration.

13. Dispositif selon au moins l'une des revendications 1 à 12, **caractérisé en ce que**, en se fondant sur l'analyse, un résultat d'analyse est généré, qui renseigne sur la nature et la constitution des voies respiratoires hautes.

14. Dispositif selon au moins l'une des revendications 1 à 13, **caractérisé en ce qu'**une observation de l'évolution de la forme de courbe s'effectue, laquelle comprend l'analyse du nombre de maxima et de minima locaux, de l'amplitude des maxima et minima locaux, de la série des ampleurs des amplitudes des maxima et minima locaux ainsi que de la fréquence dans la série des maxima et minima locaux.

15. Dispositif selon au moins l'une des revendications 1 à 14, **caractérisé en ce que** le traitement de signal comprend également l'observation spectrale et de la valeur en amplitude d'un signal de ronflement.

16. Dispositif selon au moins l'une des revendications 1 à 15, **caractérisé en ce que** l'analyse de traitement de signal et l'analyse de tendance basée sur celle-ci s'effectuent en se fondant sur une observation combinée d'au moins deux paramètres indiqués ci-après : temps d'inspiration, temps d'expiration, durée de la respiration, fréquence respiratoire, volume respiratoire pendant le cycle d'inspiration, volume respiratoire pendant le cycle d'expiration, premier différentiel et deuxième différentiel du débit respiratoire, amplitudes des maxima locaux et des minima locaux, fréquence des maxima locaux et des minima locaux, points de basculement, débit d'inspiration maximal et débit d'expiration maximal.

17. Dispositif selon au moins l'une des revendications 1 à 16, **caractérisé en ce qu'**une analyse de tendance se base sur une observation de la modification des rapports d'au moins deux des paramètres indiqués ci-après : temps d'inspiration (Ix), temps d'expiration (Ex), durée de la respiration, fréquence respiratoire, volume respiratoire pendant le cycle d'inspiration, volume respiratoire pendant le cycle d'expiration, premier différentiel et deuxième différentiel du débit respiratoire, amplitudes des maxima locaux et des minima locaux, fréquence des maxima locaux et des minima locaux, points de basculement, débit d'inspiration maximal et débit d'expiration maximal.

18. Dispositif selon au moins l'une des revendications 1 à 17, **caractérisé en ce que**, en se fondant sur l'analyse, des résultats d'analyse sont générés, qui renseignent sur :
- la nature des voies respiratoires hautes, entre autres pour la différenciation entre des apnées centrales et obstructives,
- les propriétés élastiques des voies respiratoires hautes (module de rappel, module d'élasticité)
- le lieu d'une obstruction
- le degré de sévérité d'une apnée du sommeil
- la valeur Pcrit.

19. Dispositif selon l'une des revendications précédentes, dans lequel le système de traitement de signal génère, en se fondant sur une modification de caractéristiques de référence spécifiques d'un cycle respiratoire, un résultat d'analyse qui indique si ou dans quelle mesure un trouble respiratoire prédominant ou préexistant est d'origine obstructive ou centrale et dans lequel la valeur théorique est déterminée en prenant en compte ce résultat d'analyse.

20. Dispositif selon la revendication 19, **caractérisé en ce que** celui-ci comprend une conduite de gaz respiratoire s'étendant entre le système d'acheminement et un masque respiratoire, et un système de masque respiratoire.
